# DEMANDE DE BREVET EUROPEEN

(11) **EP 1 661 576 A1**
(43) Date de publication de la demande: **31.05.2006**
(21) Numéro de dépôt: 04292713.7
(22) Date de dépôt: 15.11.2004
(51) Int. Cl.: A61K 36/45

(54) **Nouvelle utilisation dermatologique et/ou cosmétique d'un extrait végétal**

(71) Demandeur: Pharmatoka SAS, 92563 Rueil Malmaison Cédex (FR)
(72) Inventeur: Renard, Loic, 92563 Rueil Malmaison (FR); Elaers, Gunther, 92563 Rueil Malmaison (FR)
(74) Mandataire: Burtin, Jean-François

(57) **Abrégé**

L'invention se rapporte au domaine des nécessités de la vie, et plus particulièrement au domaine des soins de la peau.

Elle a plus particulièrement pour objet de nouvelles compositions dermatologiques et/ou cosmétiques, caractérisées en ce qu'elles renferment, à titre d'ingrédient actif, un extrait de Cranberry (Vaccinium macrocarpon)qui est la Grande Airelle rouge d'Amérique du nord, appelée également Canneberge au Canada, dénommée ci-après comme extrait de Cranberry (Vaccinium macrocarpon), en association ou en mélange avec un excipient ou un véhicule solide, semi fluide ou fluide, adapté pour l'application sur la peau, sur les phanères ou sur les muqueuses.

Les préparations selon l'invention renferment de 0,2 à 10 % extrait de Cranberry (Vaccinium macrocarpon) en poids de la préparation totale.

Utilisation des compositions selon l'invention en tant qu'agent tenseur de la peau, comme agent anti-oxydant ou comme agent anti-bactérien, ou comme agent cicatrisant.

## Description

La présente invention se rapporte au domaine des nécessités de la vie et, plus particulièrement, au domaine des soins de la peau.

La présente invention concerne plus précisément de nouvelles compositions dermatologiques et/ou cosmétiques, à base d'un extrait végétal manifestant des propriétés anti-oxydantes et/ou tensives et/ou anti-bactériennes.

La présente invention concerne spécifiquement de nouvelles compositions dermatologiques et/ou cosmétiques caractérisées en ce qu'elles renferment à titre d'ingrédient actif, un extrait de Cranberry (Vaccinium macrocarpon) en association ou en mélange avec un excipient ou un véhicule adapté pour l'application sur la peau ou les phanères.

Les extraits de Cranberry (Vaccinium macrocarpon) sont déjà utilisés comme agents anti-bactériens, et notamment comme agents inhibant l'adhésion des corps microbiens dans les différents tractus de l'organisme, ainsi qu'il est décrit dans la demande de brevet français, numéro 04-09526 du 8 septembre 2004. Ils sont également utiles pour imprégner les sondes et les cathéters, les canules, pour éviter ou réduire la survenue d'infections nosocomiales, fréquentes dans les hôpitaux ou les cliniques après intervention chirurgicale. Les extraits de Cranberry (Vaccinium macrocarpon) trouvent ainsi un emploi très important dans la prévention ou le traitement des infections urinaires, notamment lors de l'implantation d'une sonde à demeure, ou pendant le temps d'un examen.

La présente invention a un tout autre objet : il s'agit de l'utilisation des extraits de Cranberry (Vaccinium macrocarpon) dans des préparations topiques, en tant qu'agent tenseur de la peau, comme agent anti-oxydant ou encore comme agent anti-bactérien destiné à éliminer de la peau et/ou des téguments et/ou des muqueuses les germes microbiens susceptibles d'entraîner des manifestations microbiennes à répétition. Ceci concerne, en particulier, les staphylococcies cutanées, les furonculoses, l'anthrax, et surtout l'acné juvénile, ainsi que les infections microbiennes de la sphère bucco-dentaire. Les extraits de Vaccinium macrocarpon trouvent également une utilisation précieuse dans le cas des infections à streptocoques, qui sont souvent des infections récidivantes.

Les extraits de Cranberry (Vaccinium macrocarpon) peuvent, de ce fait, trouver un emploi sous forme de préparations solides, comme des poudres, des préparations semi fluides, comme des pâtes, ou des préparations fluides comme des crèmes, des gels fluides, des laits, des lotions ou des shampooings, ou encore des solutions, des dispersions ou des émulsions.

D'une manière plus commode, les préparations topiques se présentent sous forme d'émulsion huile dans l'eau ou eau dans l'huile, de manière à présenter une grande possibilité d'application.

Dans les compositions selon l'invention, la teneur en extrait de Cranberry (Vaccinium macrocarpon) varie de 0,2 à 10 % en poids, et de préférence de 0,5 à 4 % en poids de la préparation totale.

A cette fin, les extraits de Cranberry (Vaccinium macrocarpon) seront dissous, ou dispersés, dans une phase aqueuse, et celle-ci sera dispersée dans une phase huileuse, comme une huile végétale, additionnée d'un agent dispersant, tel qu'un agent tensioactif anionique ou non ionique, de façon à obtenir une émulsion fine, parfaitement stable.

Il est également possible de disperser les extraits de Vaccinium macrocarpon dans une phase huileuse éventuellement gélifiée par addition d'une gomme végétale comme une gomme Guar, la gomme Géllane, la gomme xanthane ou un alginate, puis de répartir cette préparation huileuse dans une phase aqueuse pour réaliser une crème, un gel, ou une émulsion huile dans l'eau.

L'extrait de Vaccinium macrocarpon est un extrait aqueux, éventuellement concentré ou reconstitué après atomisation de la poudre d'extrait déshydraté. Les extraits de Cranberry (Vaccinium macrocarpon) se caractérisent par une teneur élevée en polyphénols et, en particulier, par la présence de proanthocyanidines. Les extraits selon l'invention sont des produits commerciaux qui peuvent être employés directement dans les préparations, et notamment dans les préparations topiques selon l'invention, ou bien alors additionnés d'agents antiseptiques ou anti-bactériens qui renforcent sont efficacité. On citera, à cet égard, la chlorhexidine et ses sels, ainsi que l'hexamidine.

Les préparations topiques selon l'invention peuvent contenir, en outre :
- des agents gélifiants,
- des agents émollients,
- des agents adoucissants,
- des agents fluidifiants,
- des agents facilitant l'adhésion ou l'application,
- des agents qui améliorent le toucher lors de l'application,
- des agents aromatisants,
- ou des agents opacifiants.
   - Parmi les agents gélifiants, on citera, en particulier, les gommes végétales ou les polymères d'acide acrylique ou d'acrylamide, comme les carbomères ;
   - Parmi les agents adoucissants, on citera en particulier les polyéthylène-glycols, comme par exemple le Macrogol 1500 ou le Macrogol 4000 ;
   - Parmi les agents fluidifiants, on citera le glycérol, les glycols, le butanediol, le propylène-glycol et leurs polymères ;
   - Parmi les agents qui favorisent l'adhésion, on citera les silices colloïdales ou le dioxyde de titane micronisé ;
   - Parmi les substances qui améliorent le toucher, on citera plus particulièrement les silicones et, en particulier, les huiles de silicone, la diméthicone, la siméthicone et l'hexadiméthicone ;
   - Parmi les agents aromatisants, on citera particulièrement l'essence de lavande, l'essence de petit grain, l'essence de néroli, l'essence de géranium Rosat, l'essence de lavandin, l'essence d'ylang-ylang, l'essence de menthe, ou l'essence de pamplemousse ;
   - Parmi les agents opacifiants, on citera plus particulièrement les stéarates métalliques, et, en particulier, le stéarate de calcium, le stéarate de magnésium, l'oxyde de Zinc, le carbonate de Calcium, le talc, l'alumine, les silicates d'aluminium.

Comme indiqué précédemment, les préparations topiques selon l'invention peuvent refermer des concentrations variables d'extrait de Vaccinium macrocarpon. Un extrait particulièrement utilisé est celui qui contient de 60 à 120 mg de proanthocyanidines pour 10 ml de concentré de jus. Cet extrait peut être incorporé dans les préparations topiques, à raison de 10 à 50 ml pour 100 ml d'excipient solide ou liquide ou fluide, en vue de la réalisation d'une crème, d'un gel, d'un lait ou d'une lotion.

Ces préparations sont destinées à être appliquées à des fins dermatologiques ou cosmétiques, à raison d'une à quatre fois par jour.

La durée du traitement sera en fonction des besoins de la peau. L'utilisation comme agent tenseur de la peau nécessite une utilisation plus prolongée que l'utilisation comme anti-oxydant. L'utilisation comme agent anti-bactérien cutané ou sur les muqueuses requiert des applications plus courtes, mais souvent renouvelées. Le traitement comme adjuvant de la cicatrisation pour des plaies chroniques ou récidivantes nécessite des applications très fréquentes, notamment sous forme de pansements occlusifs ou aérés, avec des hydrocolloïdes pour aider ou faciliter la cicatrisation des plaies.

Les compositions topiques selon l'invention trouvent un emploi plus particulier comme tenseur, c'est-à-dire comme agent capable de retendre la peau, et de lui donner une nouvelle fermeté. En outre, les propriétés antiseptiques de l'extrait de Cranberry (Vaccinium macrocarpon) permettent aux préparations dermatologiques ou cosmétiques de l'invention de jouer un rôle important pour lutter contre les rougeurs ou les irritations résultant d'infections cutanées, ou de coupures, telles que celles résultant du feu du rasoir.

Les compositions topiques selon l'invention trouvent aussi un emploi comme agent antiseptique et/ou cicatrisant pour les muqueuses, dans des préparations aqueuses destinées à des bains de bouche, dans le cas d'aphtes ou d'ulcération du palais ou de la paroi buccale ou dans des préparations solides, notamment pulvérulentes.

Commodément, les bains de bouche selon l'invention renferment de l'extrait de Vaccinium macrocarpon dilué dans un véhicule aqueux et éventuellement rendu plus visqueux par l'addition de glycérol à la concentration de 0,5 à 10 g d'extrait de Vaccinium macrocarpon pour 100 mg de véhicule aqueux.

Les exemples suivants illustrent l'invention. Ils ne la limitent en aucune façon.

### EXEMPLE I : Gel tenseur de la peau, à base d'extrait de Cranberry (Vaccinium macrocarpon)

| | |
|---|---|
| Extrait de Vaccinium macrocarpon | 2,5 g |
| Cire Lanette | 0,4 g |
| Propylèneglycol | 3,0 g |
| Glycérol | 5,0 g |
| Triéthanolamine | 0,2 g |
| Polymère d'acide acrylique, commercialisé sous la dénomination CARBOPOL P 956® (Goodrich) | 0,75 g |
| Ether dicaprylique | 4,0 g |
| Germaben II (agent conservateur) | 0,70 g |
| Eau déminéralisée qsp | 100,00 g |

### EXEMPLE II : Lotion anti-bactérienne de la peau à base d'extrait de Cranberry (Vaccinium macrocarpon))

| | |
|---|---|
| Extrait de Vaccinium macrocarpon | 13,75 g |
| Isononanoate d'isononanoyle | 1,25 g |
| Cire Lanette C 16 | 0,5 g |
| Lécithine hydrogénée | 0,5 g |
| DC-1403 FLUIDE (Huile de silicone à base d'un mélange de diméthiconol et de diméthicone) | 0,25 g |
| Cétylphosphate de potassium (commercialisé sous la dénomination Amphisol K) | 1,50g |
| Parfum fleur (parfum lotus AF 3817) qs | |
| Germaben II (agent conservateur) | 0,70 g |
| Eau qsp | 1 000 ml |

### EXEMPLE III : Emulsion anti-oxydante à base d'extrait de Cranberry (Vaccinium macrocarpon)

| | |
|---|---|
| Extrait de Vaccinium macrocarpon | 3,75 g |
| Cyclométhicone | 0,4 g |
| Glycérol | 2,5 g |
| Cire lanette | 1,75 g |
| Alcool cétylique | 5,75 g |
| Stéarate de polyéthylène glycol 20 (Myrj49 de la société ICI) | 1,50g |
| Concentré de Lavande qs | |
| Eau déminéralisée qs p | 100 ml |

### EXEMPLE IV : Crème fluide à base d'extrait de Vaccinium macrocarpon

| | |
|---|---|
| Extrait fluide de Vaccinium macrocarpon | 2,50 g |
| Gamma Oryzanol | 0,25 g |
| Lécithine végétale | 0,25 g |
| Avocadine | 1,20 g |
| Squalène | 0,50 g |
| Acide stéarique | 0,60 g |
| Propylène glycol | 2,50 g |
| Phénonip | 0,50 g |
| Norgel (agent épaississant) | 0,60 g |
| Huile d'amandes douces | 7,00g |
| Eau qsp | 100 ml |

### EXEMPLE V : Tampons de gaze imprégnés d'un extrait fluide de Cranberry (Vaccinium macrocarpon)

| | |
|---|---|
| Hyaluronate de Sodium, correspondant à 210 g de produit après dessication | 220,5 g |
| Extrait de Cranberry Vaccinium macrocarpon | 125,0 g |
| Macrogol 4500 | 12,0 g |
| Glycérol | 105,0 g |
| Eau purifiée qsp | 4 200,0 g |

| | |
|---|---|
| **Pour 1 000 compresses** | |

On place, dans un réservoir thermostaté, la dispersion, préparée au préalable, d'hyaluronate de sodium et d'extrait de Vaccinium, macrocarpon. Les tampons de gaze déroulés d'une bobine, sont imprégnés pendant les passages dans le rouleau qui prélève de la dispersion. La quantité de dispersion appliquée dépend de la vitesse d'avancement du rouleau et de la température à laquelle le réservoir est thermostaté. Le réglage est effectué de façon à ce que la quantité de dispersion qui imprègne les tampons varie de 3,6 à 4,4 g par tampon.

Les tampons de gaze progressent entre deux bobines de film de polyéthylène, directement en contact avec la gaze imprégnée, puis de polypropylène. Ces tampons, ainsi revêtus, sont ensuite découpés en segments après enveloppement par assemblage de deux feuillets de papier polyéthylène-aluminium provenant de deux bobines différentes, au moyen de deux couteaux opérant immédiatement après formation d'une telle enveloppe. Après insertion du tampon de gaze dans cette enveloppe, une machine de conditionnement scelle les deux extrémités et découpe chaque enveloppe.

Les tampons de gaze imprégnée d'extrait de Vaccinium macrocarpon servent de dispositif cicatrisant.

### EXEMPLE VI : Bain de bouche à base d'extrait de Vaccinium macrocarpon

| | |
|---|---|
| Extrait fluide de Vaccinium macrocarpon | 1,50 g |
| Glycérol | 1,50 g |
| Borate de sodium | 0,60 g |
| | |
| Saccharinate de sodium | 0,02 g |
| Eau purifiée qsp | 100 ml |

## Revendications

1. Compositions dermatologiques et/ou cosmétiques et/ou mucosales,
**caractérisées en ce qu'**
elles renferment, à titre d'ingrédient actif, un extrait de Cranberry (Vaccinium macrocarpon) en association ou en mélange avec un excipient ou un véhicule adapté pour l'application sur la peau, sur les phanères ou sur les muqueuses.

2. Compositions dermatologiques et/ou cosmétiques et/ou mucosales selon la revendication 1,
dans lesquelles la teneur en extrait de Cranberry varie de 0,2 à 10 % en poids de la préparation totale.

3. Compositions dermatologiques et/ou cosmétiques selon la revendication 1 ou la revendication 2,
dans lesquelles la teneur en extrait de Cranberry varie de 0,5 à 4 % en poids de la préparation totale.

4. Compositions dermatologiques et/ou cosmétiques et/ou mucosales selon l'une des revendications précédentes,
dans lesquelles l'extrait de Cranberry est un extrait aqueux éventuellement concentré ou reconstitué.

5. Compositions dermatologiques et/ou cosmétiques et/ou mucosales selon l'une des revendications précédentes,
dans lesquelles l'extrait de Cranberry contient de 20 à 50 mg de proanthocyanosides pour 100 ml de jus.

6. Compositions dermatologiques et/ou cosmétiques et/ou mucosales selon l'une des revendications précédentes,
dans lesquelles l'extrait de Cranberry est incorporé, à raison de 1 à 50 ml pour 100 ml d'excipient solide, liquide ou fluide.

7. Compositions dermatologiques et/ou cosmétiques et/ou mucosales selon l'une des revendications précédentes,
dans lesquelles l'excipient ou le véhicule est l'un de ceux qui conviennent pour la réalisation de préparations solides, semi fluides ou fluides.

8. Compositions dermatologiques et/ou cosmétiques et/ou mucosales selon l'une des revendications précédentes,
dans lesquelles les extraits de Cranberry sont dissous ou dispersés dans une phase aqueuse qui, elle-même, est dispersée dans une phase huileuse pour obtenir une émulsion fine, parfaitement stable, eau dans l'huile.

9. Compositions dermatologiques et/ou cosmétiques et/ou mucosales selon les revendications 1 à 7,
dans lesquelles l'extrait de Cranberry est dispersé dans une phase huileuse, éventuellement gélifiée, puis on répartit cette préparation huileuse dans une phase aqueuse pour réaliser un gel, une crème ou une émulsion huile dans l'eau.

10. Compositions dermatologiques et/ou cosmétiques et/ou mucosales selon l'une des revendications précédentes,
qui contiennent, en outre, au moins l'un des agents suivants choisis parmi les agents gélifiants, les agents émollients, les agents adoucissants, les agents fluidifiants, les agents facilitant l'adhésion ou l'application, les agents qui améliorent le toucher, les agents aromatisants et les agents opacifiants.

11. Compositions dermatologiques selon la revendication 1,
dans lesquelles l'extrait de Vaccinium macrocarpon est incorporé dans un hydrocolloïde et appliqué sur des tampon de gaze que l'on dispose dans des feuillets de matériau isolant, scellé aux deux extrémités et découpé à la dimension désirée.

12. Compositions pharmaceutiques destinées à l'application sur les muqueuses, et notamment sur la muqueuse de la bouche et le palais,
dans lesquelles l'extrait de Vaccinium macrocarpon est dissout ou dispersé dans un véhicule aqueux additionné éventuellement d'un agent viscosifiant, d'un agent aromatisant, d'un agent antiseptique et/ou d'un agent édulcorant.

## Revendications modifiées

### Revendications modifiées conformément à la règle 86(2) CBE.

**1.** Utilisation comme agent anti-oxydant et/ou comme agent de cicatrisation et/ou pour éliminer les germes microbiens de la peau et /ou des téguments et/ou des muqueuses
d' un extrait aqueux de Cranberry (Vaccinium macrocarpon), éventuellement concentré ou reconstitué.

**2.** Utilisation selon la revendication 1, **caractérisée par le fait que** l'extrait de Cranberry est incorporé dans des compositions dermatologiques et/ou cosmétiques et/ou mucosales, en association ou en mélange avec un excipient ou un véhicule adapté pour l'application sur la peau, sur les phanères ou sur les muqueuses.

**3.** Utilisation selon l'une des revendications 1 ou 2, **caractérisée par le fait que** la teneur en extrait de Cranberry des compositions dermatologiques et/ou cosmétiques et/ou mucosales, varie de 0,2 à 10 % en poids de la préparation totale.

**4.** Utilisation selon l'une des revendications précédentes, **caractérisée par le fait que** la teneur en extrait de Cranberry des compositions dermatologiques et/ou cosmétiques et/ou mucosales varie de 0,5 à 4 % en poids de la préparation totale.

**5.** Utilisation selon l'une des revendications précédentes, **caractérisée par le fait que** l'extrait de Cranberry des compositions dermatologiques et/ou cosmétiques et/ou mucosales contient de 20 à 50 mg de proanthocyanosides pour 100 ml de jus.

**6.** Utilisation selon l'une des revendications précédentes, **caractérisée par le fait que** l'extrait de Cranberry est incorporé, à raison de 1 à 50 ml pour 100 ml d'excipient solide, liquide ou fluide dans des compositions dermatologiques et/ou cosmétiques et/ou mucosales.

**7.** Utilisation selon l'une des revendications précédentes, **caractérisée par le fait que** l'excipient ou le véhicule des compositions dermatologiques et/ou cosmétiques et/ou mucosales est l'un de ceux qui conviennent pour la réalisation de préparations solides, semi fluides ou fluides.

**8.** Compositions dermatologiques et/ou cosmétiques et/ou mucosales, destinées à être utilisées comme agent anti-oxydant et/ou comme agent de cicatrisation et/ou pour éliminer les germes microbiens de la peau et /ou des téguments et/ou des muqueuses
**caractérisées par le fait qu'**un extrait aqueux de Cranberry est dissous ou dispersé dans une phase aqueuse qui, elle-même, est dispersée dans une phase huileuse pour obtenir une émulsion fine, parfaitement stable, eau dans l'huile.

**9.** Compositions dermatologiques et/ou cosmétiques et/ou mucosales, destinées à être utilisées comme agent anti-oxydant et/ou comme agent de cicatrisation et/ou pour éliminer les germes microbiens de la peau et /ou des téguments et/ou des muqueuses,
**caractérisées par le fait qu'**un extrait aqueux de Cranberry est dispersé dans une phase huileuse, éventuellement gélifiée, puis cette préparation huileuse est répartie dans une phase aqueuse pour réaliser un gel, une crème ou une émulsion huile dans l'eau.

**10.** Compositions selon l'une des revendications 8 ou 9, **caractérisées par le fait qu'**elles contiennent, en outre, au moins l'un des agents suivants choisis parmi les agents gélifiants, les agents émollients, les agents adoucissants, les agents fluidifiants, les agents facilitant l'adhésion ou l'application, les agents qui améliorent le toucher, les agents aromatisants et les agents opacifiants.

**11.** Utilisation selon l'une des revendications 1 ou 2, **caractérisée par le fait que** l'extrait de Vaccinium macrocarpon est incorporé dans un hydrocolloïde et appliqué sur des tampon de gaze que l'on dispose dans des feuillets de matériau isolant, scellé aux deux extrémités et découpé à la dimension désirée.

**12.** Compositions destinées à être utilisées comme agent anti-oxydant et/ou comme agent de cicatrisation et/ou pour éliminer les germes microbiens, par application sur les muqueuses, et notamment sur la muqueuse de la bouche et sur le palais,
dans lesquelles un extrait aqueux de Vaccinium macrocarpon est dissout ou dispersé dans un véhicule aqueux additionné éventuellement d'un agent viscosifiant, d'un agent aromatisant, d'un agent antiseptique et/ou d'un agent édulcorant.
